Europäisches Patentamt

European Patent Office

Office européen des brevets

(11) Publication number: **0 376 448 B1**

# EUROPEAN PATENT SPECIFICATION

(45) Date of publication of patent specification: **14.07.93**  (51) Int. Cl.⁵: **A61L  9/01**

(21) Application number: **89311296.1**

(22) Date of filing: **01.11.89**

(54) **Deodorizer composition.**

(30) Priority: **29.12.88 JP 334932/88**
**29.06.89 JP 169351/88**

(43) Date of publication of application:
**04.07.90 Bulletin  90/27**

(45) Publication of the grant of the patent:
**14.07.93 Bulletin  93/28**

(84) Designated Contracting States:
**GB**

(56) References cited:
EP-A- 0 201 209    EP-A- 0 282 287
WO-A-81/01643      FR-A- 1 428 096
FR-A- 2 485 034    GB-A- 895 683

(73) Proprietor: **LION CORPORATION**
**3-7, Honjo 1-chome**
**Sumida-ku Tokyo(JP)**

(72) Inventor: **Miyano, Masakazu**
**2-32-408, Ayamedai**
**Chiba-shi Chiba(JP)**
Inventor: **Ueno, Akira**
**5-21-403, Takatsu-danchi**
**Yachiyo-shi Chiba(JP)**
Inventor: **Nakajyu, Isamu**
**3-31-17, Hiyoshidai Tomisato-cho**
**Inba-gun Chiba(JP)**
Inventor: **Sato, Teiji**
**3-25, Shintomi-cho 3-chome**
**Shibata-shi Niigata(JP)**

(74) Representative: **Adams, William Gordon et al**
**RAWORTH, MOSS & COOK 36 Sydenham**
**Road**
**Croydon Surrey CR0 2EF (GB)**

Rank Xerox (UK) Business Services
(3. 10/3.6/3.3. 1)

**Description**

BACKGROUND OF THE INVENTION

1. Field of the Invention

The present invention relates to a deodorizer composition, more specifically, to a deodorizer composition having a strong deodorizing power which can be widely utilized to eliminate objectionable odors in the home and mitigate objectionable industrial odors generated by factories and the like.

2. Description of the Related Art

The increasing urbantization of formerly sparsely-populated areas has brought more people into daily contact with the various smells and odors of their environment, and this has led to a strong interest in, and severe criticism of, particularly offensive odors. Various methods of treating offensive odors are known in the art, of which the following are typical:

(1) Sensitive Deodorizing ... masking with perfumes or flavors;

(2) Physical Deodorizing ... adsorption with, for example, activated carbon or absorption or clathrating with, for example, cyclodextrin;

(3) Chemical Deodorizing ... neutralization with an acid or alkali, oxidation or reduction with an oxidizing agent or reducing agent, or an addition of, for example, lauryl methacrate; and

(4) Biological Deodorizing ... deodorizing by germicidal action with germicide, or effects obtained with microorganisms or enzymes.

The sensitive deodorizing method is often unsuccessful since only the flavor of the odor is changed and the offensive odor itself is still present although masked, and thus if the balance between the offensive odor and the flavor is lost, the offensive odor again prevails and the balance must be restored.

In the physical deodorizing method, although the offensive odor is absorped or clathrated, problems arise in that the adsorption power or clathrating power is not strong enough for practical use.

In the chemical deodorizing method, certain chemicals should not be used from the viewpoint of safety, and although a chemical deodorization of one offensive odor can be achieved, this same process will have no affect on the other various offensive odors generated in daily life.

In the biological deodorizing method, disadvantages in the deodorizing rate and a continuous effect or durability arise and, therefore, universal effects cannot be obtained by a single deodorizing method.

Conventionally activated carbon is most widely used, and is known as a deodorizer capable of adsorbing various offensive odor components. Nevertheless, among such offensive odor components, the capability of activated carbon for the adsorption of lower amines is small, and in particular, the deodorizing power thereof against hydrogen sulfide and ammonia is low.

Various attempts have been made to solve the above-mentioned problems. For example, JP-A-55-51421 (i.e., Japanese Unexamined Patent Publication) proposes that halides be supported on activated carbon; JP-A-53-137089 proposes that metals be supported on activated carbon; and the adhesion or deposition of acids or alkalis has been studied. Nevertheless, these proposals are not still satisfactory.

Furthermore, although the above-mentioned patent publications generally describe deodorizing performances against various objectionable or offensive odors, the deodorizing speed (or rate) and the deodorizing amounts are also important factors, taking into consideration the utility of the deodorizer compositions.

The present inventors have proposed, in JP-A-63-220874, the use of aluminosilicates having a specified $SiO_2$, $MO_{n/2}$ and $Al_2O_3$ composition in terms of the oxides expressed by the three component composition as a deodorizer.

SUMMARY OF THE INVENTION

Accordingly, the objects of the present invention are to eliminate the above-mentioned disadvantages of the prior art and to provide a deodorizer composition exhibiting excellent deodorizing effects for various objectionable odors, and having a high deodorizing speed and safety.

Another object of the present invention is to provide a deodorizer composition exhibiting an excellent deodorizing effect for various objectionable odors, and an excellent persistency of the effect.

Other objects and advantages of the present invention will be apparent from the following description.

In accordance with the present invention, there is provided a deodorizer composition comprising:

(a) an aluminosilicate having the following composition, in terms of oxides, represented by the composition ratio of the three components:

$SiO_2$ : 5 to 80 mole%;

$MO_{n/2}$ : 5 to 65 mole%;

$Al_2O_3$ : 1 to 60 mole%;

wherein M represents at least one metal selected from the group consisting of zinc, copper, silver, cobalt, nickel, iron, titanium, barium, tin and zirconium, and n represented a valence of metal; and

(b) at least one component selected from the group consisting of (i) at least one oxidizing agent selected from the group consisting of organic peracids and salts thereof and persulfate, (ii) at least one plant extract selected from the group consisting of Rosemary extracts and Sage extracts, and (iii) at least one germicide selected from the group consisting of p-chloro-m-xylenol, butyl-m-cresol, o-phenylphenol, and $\alpha$-bromocinnamic aldehyde.

## DESCRIPTION OF THE PREFERED EMBODIMENT

The activated charcoal, silica gel, clay mineral, and aluminosylycate usable as the component (a) in the present invention preferably has a BET specific area of 100 $m^2/g$ or more, more preferably 500 to 2000 $m^2/g$, when determined with $N_2$ gas.

Activated charcoal may be obtained by a treatment of, for example, coal, petroleum residue, charcoal, fruit, and shells, by the gas activation method using, for example, steam, carbon dioxide, or the chemical activation method using, for example, zinc chloride.

As the aluminosilicate, zeolite and those having the composition ratio of the three components represented in terms of oxides shown below are preferable:

$SiO_2$: 5 to 80 mole%, preferably 25 to 75 mole%;

$Mo_{n/2}$: 5 to 65 mole%, preferably 15 to 60 mole%;

$Al_2O_3$: 1 to 60 mole%, preferably 1 to 45 mole%

wherein M represents at least one metal selected from zinc, copper, silver, cobalt, nickel, iron, titanium, barium, tin and zirconium, and n represents a valence of metal.

Of the component (a), aluminosilicates, in particular are considered to have the characteristics of a solid acid and solid acid group, and it has been reported that the acidity varies depending on the content of alumina in silica-alumina type catalysts, and that the acidity differs depending on the ratio of the contents of alumina and silica (Kozo Okabe: Acidic and Basic Catalysts, p. 183 (1967)). Further, since the aluminosilicate has a structure in which acidic $SiO_2$ and basic metal oxides are bound together, it has basic and acidic polarities and may be considered to exhibit deodorizing effect for objectionable odor components based primarily on a chemical adsorption and a chemical reaction.

The aluminosilicate is obtained as a white or pale-colored powder, and is prepared by allowing a water soluble silicate, a water-soluble metal salt, and further, a water-soluble salt aluminum salt and/or a water-soluble aluminate, in amounts corresponding to the above-mentioned composition ratio, to react in the presence of water, and if necessary, heating the precipitates obtained in the presence of water.

The above-mentioned reaction proceeds steadily due to a metathetical reaction. Namely, when an alkali silicate such as sodium silicate is used as a silica component, a water-soluble metal salt such as a chloride, nitrate, and sulfate is used as a metal oxide component, and further, sodium aluminate and/or a water-soluble aluminum salt such as aluminum chloride or aluminum sulfate are used as the alumina component, these components are mixed together in the presence of water, followed by effecting the metathetical reaction.

To uniformly effect the above-mentioned metathetical reactions, it is preferable to simultaneously add, to an aqueous dispersion containing silica previously dispersed therein, an aqueous silicate solution, an aqueous metal salt solution and an aqueous solution containing the alumina component, while allowing a reaction.

The above-mentioned metathetical reaction can be carried out at room temperature but, of course, also can be carried out upon heating at a temperature of up to, for example, about 95°C.

The preferable pH range of the reaction during the simultaneous addition is 5 to 10, most preferably especially 6 to 9. If necessary, the pH of the reaction mixture can be controlled by the addition of an acid or alkali, to maintain the above-mentioned pH range.

The above-mentioned simultaneous addition of the reactants allows the formation of precipitated aluminosilicate having a composition substantially corresponding to that of the aqueous solution. The precipitates thus formed are separated, or optionally, are heated in the presence of water, to obtain a white or pale-colored fine powder.

As the oxidizing agent (i) of the component (b), potassium persulfate, potassium monopersulfate, sodium persulfate, ammonium persulfate, diperoxydodecane diacid, and magnesium perfumarate, are included as representative examples. Among the above, persulfates such as sodium persulfate, potassium persulfate, ammonium persulfate, and potassium monopersulfate; organic peracids such as diperoxydodecane diacid, and magnesium perfumarate, and salts thereof, are preferred.

As the plant extract (ii) of the component (b), an extract of Salvia officinalis L. and Rosmarinus officinalis L.,

To obtain solvent extracts from these plants, as the plant, either the whole grass or the respective sections of plants such as leaves, plant skin, flowers, fruit skin, fruit, root stalk, or root may be employed, and the section containing relatively more of the deodorizing effective ingredient of the plant can be selected.

When obtaining deodorizing effective ingredients from these plants, known methods can be employed. For example, it is possible to employ the method in which plants dried and then cut and formed into powder, are extracted with at least one polar solvent such as water, ethyl ether, ethylene chloride, dioxane, acetone, ethanol, n-butanol, ethyl acetate, propylene glycol or at least one nonpolar solvent such as n-hexane, petroleum ether, ligroin, cyclohexane, carbon tetrachloride, chloroform, dichloromethane, 1,2-dichloroethane, toluene, benzene, or a mixed solvent of these polar solvents and nonpolar solvents. In this case, as the extraction operation, a conventional method can be employed in which, for example, plants are subjected to maceration in a solvent.

The extract obtained by the extraction operation as described above is usually formulated in a deodorizer after evaporation of the solvent, but in some cases, particularly when the solvent is water, ethanol can be also formulated as such in a deodorizer without evaporation of the solvent.

Examples of the germicide (iii) of the component (c) include p-chloro-m-xylenol, butyl-m-cresol, o-phenylpheol and $\alpha$-bromocinnamic aldehyde are preferred.

The deodorizer composition of the present invention can be obtained with the above components (a) and (b) as the essential components.

These respective components may be formed into powdery or granular products and mixed, the component (a) may be impregnated with the component (b), and a carrier may be impregnated or attached with the component (b) and mixed with the component (a), or further, the components (a) and (b) may be kneaded and granulated under the condition maintained in a solvent.

In the deodorizer composition of the present invention, it is preferable to contain the component (a) and the component (b) at a weight ratio of (a)/(b) = 1/99 to 99.99/0.01, more preferably 50/50 to 99.9/0.1, especially 50/50 to 99.5/0.5. Also, if necessary, optional components can be further formulated in the composition.

In the composition of the present invention, in addition to the components (a) and (b), optional components such as surfactants, alkali agents, acid substances, dyes, UV-ray absorbers, antioxidants, and polymeric substances can be also formulated, if necessary.

A gas having an objectionable odor generally consists of a large number of components. As representative objectionable odor components, nitrogen type gases such as ammonia and amine, and sulfur type gases such as hydrogen sulfide and mercaptans, are known and are generated in large amounts. Nevertheless, since these nitrogen type and sulfur type objectionable odor components differ in behavior, an effective deodorizing base material for both objectionable odor components was not known.

In the present invention, by using the component (a) and the component (b), it is now possible to deodorize various objectionable odors. The performances demanded of deodorizers include, in addition to effects against various objectionable odors, a rapid deodorizing speed and large deodorizing capacity, i.e., long-term persistency.

In the present invention, the component (a), provides a deodorizing effect against various objectionable odors, and further, by using the component (b) in combination therewith, the deodorizing speed and the deodorizing capacity are improved.

Of the porous substances of the component (a), aluminosilicates in particular are considered to have the characteristics of a solid acid and solid acid group, and it has been reported that the acidity varies depending on the content of alumina in silica-alumina type catalysts, and that the acidity differs depending on the ratio of the contents of alumina and silica [Kozo Okabe: Acid Base Catalyst, p. 183 (1967)]. Further, since the aluminosilicate has a structure in which acidic $SiO_2$ and basic metal oxides are bound together, it has basic and acidic polarities and is considered to exhibit a deodorizing effect against objectionable odor components based primarily on a chemical adsorption and chemical reaction.

On the other hand, porous substances having a specific surface area of 100 $m^2/g$ or more as represented by activated charcoal have an excellent physical adsorption.

The deodorizing mechanism in the deodorizer composition of the present invention has not been defined, but it is considered that the physical adsorption proceeds at a very rapid speed until reaching an equilibrium, and subsequently, a chemical reaction occurs. That is, by combining a porous substance of the component (a) and the component (b), a rapid adsorption of objectionable odor onto a porous substance with a large specific area, and a chemical reaction of the adsorbed objectionable odor with the component (b), are considered to act synergetically, thereby effectively supplying a stronger deodorization effect.

Also, the objectionable odor trapped by the adsorption and chemical reaction will not be released as an odor, and it is considered that a release of an odor, which is a drawback of a deodorization by physical adsorption, is inhibited to improve the durability of the deodorizing effect.

According to the deodorizer composition of the present invention, by using the component (a) and an oxidizing agent (i) as the component (b) in combination, a stronger deodorizing power can be exhibited than in the case where each is used alone, but the deodorizing effect can be exhibited more rapidly and the persistency of the effect is prolonged.

In the present invention, by using the component (a), a plant extract (ii), and/or a germicide (iii) as the component (b) in combination, it is now possible to deodorize various objectionable odors.

Also, by using a plant extract (ii) of the component (b) in combination, in addition to eliminating various objectionable odors, a deodorizer composition is realized which has a rapid deodorizing speed, large deodorizing capacity, and a high safety factor.

Further, by combining a germicide (iii) of the component (b) with the component (a), the deodorizing speed and the deodorizing capacity are improved to provide a deodorizer having a prolonged persistency.

Therefore, although various objectionable odor components are exuded from the human body, in the home, and by industry facilities, the deodorizer composition of the present invention rapidly eliminates these objectionable odors and provides a long-term deodorizing effect, and thus can be widely utilized.

EXAMPLE

The present invention will now be further, illustrated by, but is by no means limited to, the following synthetic Examples of the alumino silicates, Examples of the deodorizer compositions, and results of the evaluation thereof.

Synthetic Example 1

A 109 g amount of #3 sodium silicate (SiO$_2$: 22.0%, Na$_2$O: 7.0%) and 94 g of sodium hydroxide (NaOH content: 2.35 mole) were dissolved in water to prepare 1 liter of a solution A (SiO$_2$ content: 0.4 mole). Further, 95 g of zinc chloride (anhydrous salt) and 97 g of aluminum chloride (hexahydrate) were dissolved in water to prepare 1 liter of a solution B (ZnO content: 0.7 mole, Al$_2$O$_3$: 0.2 mole).

Then, one liter of water was charged into a 5 liter beaker and, while stirring, the solutions A and B were simultaneously added thereto at a feed rate of about 25 cc/min, respectively. The pH of the resultant reaction mixture was 6.9 after the addition.

The stirring was further continued, and after aging for 30 minutes, the mixture was heated at a temperature of 85 to 90°C for 2 hours on a water bath. The reaction mixture thus obtained was filtered with aspiration, followed by washing with water and drying at a temperature of 110°C, and the cake thus obtained was sifted through a sieve to obtain zinc aluminosilicate in the form of white particles having a size of 8 to 16 mesh (Tylor).

The composition of the three components and the BET specific surface of the resultant particles are shown in Table 2, together with the data of the following Synthetic Examples.

Synthetic Example 2

In the same manner as in Synthetic Example 1, 139 g of #3 sodium silicate (SiO$_2$: 22.0%, Na$_2$O: 7.0%) and 88 g of sodium hydroxide (NaOH content: 2.2 mole) were dissolved in water to prepare 1 liter of a solution A (SiO$_2$ content: 0.51 mole). Further, 65 g of zinc chloride (anhydrous salt) and 126 g of aluminum chloride (hexahydrate) were dissolved in water to prepare 1 liter of a solution B (ZnO content: 0.48 mole, Al$_2$O$_3$: 0.26 mole).

Then, one liter of water was charged into a 5 liter beaker and, while stirring, the solutions A and B were simultaneously added thereto at a feed rate of about 25 cc/min, respectively. The pH of the resultant reaction mixture was 8.6 after the addition.

The reaction mixture obtained above was treated in the same manner as in Synthetic Example 1, whereby zinc aluminum silicate in the form of particles having a size of 8 to 16 mesh was obtained.

Synthetic Example 3

A 273 g amount of #3 sodium silicate ($SiO_2$: 22.0%, $Na_2O$: 7.0%) and 60 g of sodium hydroxide (NaOH content: 1.5 mole) were dissolved in water to prepare 1 liter of a solution A ($SiO_2$ content: 1.0 mole). Further, 34 g of silver nitrate and 225 g of aluminum nitrate (nonahydrate) were dissolved in water to prepare 1 liter of a solution B ($Ag_2O$ content: 0.1 mole, $Al_2O_3$ content: 0.3 mole).

Then, one liter of water was charged into a 5 liter beaker and, while stirring, the solutions A and B were simultaneously added thereto at a feed rate of about 25 cc/min, respectively. The pH of the resultant reaction mixture was 8.9 after the addition.

The stirring was further continued, and after aging for 1 hour, the cake thus obtained was sifted through a sieve to obtain silver aluminosilicate in the form of particles having a size of 8 to 16 mesh.

Synthetic Example 4

A 77 g amount of #1 sodium silicate ($SiO_2$: 35.0%, $Na_2O$: 17.5%) and 24 g of sodium hydroxide (NaOH content: 0.6 mole) were dissolved in water to prepare 1 liter of a solution A ($SiO_2$ content: 0.45 mole). Further, 216 g of zinc sulfate (heptahydrate) and 75 g of sodium aluminate ($Al_2O_3$ content: 20.5%, $Na_2O$ content: 19.5%) were dissolved in water to prepare 1 liter of a solution B (ZnO content: 0.75 mole, $Al_2O_3$: 0.15 mole).

Then, one liter of water was charged into a 5 liter beaker and, while stirring, the liquids A and B were simultaneously added thereto at a feed rate of about 25 cc/min, respectively. The pH of the resultant reaction mixture was 6.9 after the addition.

The stirring was further continued, and after aging for 30 minutes, the mixture was heated at a temperature of 85 to 90°C for 2 hours on a water bath. The reaction mixture thus obtained was filtered with aspiration, followed by washing with water and drying at a temperature of 110°C, and the cake thus obtained was sifted through a sieve to obtain zinc aluminosilicate in the form of white particles having a size of 8 to 16 mesh.

Synthetic Examples 5 to 12

In the same manner as in Synthetic Example 3 (silver salt), aluminosilicates containing cobalt, nickel, iron, copper, titanium, barium, tin, and zirconium were obtained from the solutions A and B listed in Table 1.

EP 0 376 448 B1

Table 1

| Compound | Synthetic Example No. | | | | | | | |
|---|---|---|---|---|---|---|---|---|
| | 5 | 6 | 7 | 8 | 9 | 10 | 11 | 12 |
| **Solution A** | | | | | | | | |
| #3 Sodium silicate (mole $SiO_2$) | 273 g (1.0) | 273 g (1.0) | 273 g (1.0) | 273 g (1.0) | 273 g (1.0) | 273 g (1.0) | 273 g (1.0) | 273 g (1.0) |
| Sodium hydroxide (mole NaOH) | 60 g (1.5) | 60 g (1.5) | 60 g (1.5) | 60 g (1.5) | 60 g (1.5) | 60 g (1.5) | 60 g (1.5) | 60 g (1.5) |
| **Solution B** | | | | | | | | |
| Aluminum chloride·$6H_2O$ (mole $Al_2O_3$) | 145 g (0.3) | 145 g (0.3) | 145 g (0.3) | 145 g (0.3) | 145 g (0.3) | 145 g (0.3) | 145 g (0.3) | 145 g (0.3) |
| Cobalt chloride (mole CoO) | 65 g (0.5) | - | - | - | - | - | - | - |
| Nickel chloride·$6H_2O$ (mole NiO) | - | 120 g (0.5) | - | - | - | - | - | - |
| Ferric nitrate·$9H_2O$ (mole FeO) | - | - | 200 g (0.5) | - | - | - | - | - |
| Copper chloride (mole CuO) | - | - | - | 67 g (0.5) | - | - | - | - |
| Titanium sulfate (mole $TiO_2$) | - | - | - | - | 120 g (0.5) | - | - | - |
| Barium chloride·$2H_2O$ (mole BaO) | - | - | - | - | - | 122 g (0.5) | - | - |
| Tin sulfate·$2H_2O$ (mole SnO) | - | - | - | - | - | - | 126 g (0.5) | - |
| Zirconium chloride (mole $ZrO_2$) | - | - | - | - | - | - | - | 116.5 g (0.5) |

Table 2

| No. | Composition Ratio of Three Components | | | BET Specific Surface Area $(m^2/g)$ |
|---|---|---|---|---|
| | $SiO_2$ (mole%) | $MO_{\frac{n}{2}}$ (mole%) | $Al_2O_3$ (mole%) | |
| Synthetic Example 1 | 31 | 54 | 15 | 210 |
| " 2 | 41 | 38 | 21 | 205 |
| " 3 | 67 | 13 | 20 | 216 |
| " 4 | 33 | 56 | 11 | 218 |
| " 5 | 55 | 28 | 17 | 185 |
| " 6 | 55 | 28 | 17 | 162 |
| " 7 | 55 | 28 | 17 | 158 |
| " 8 | 55 | 28 | 17 | 163 |
| " 9 | 55 | 28 | 17 | 171 |
| " 10 | 55 | 28 | 17 | 160 |
| " 11 | 55 | 28 | 17 | 154 |
| " 12 | 55 | 28 | 17 | 172 |

Example 1

Each of the aluminosilicates obtained in Synthesis Examples 1 to 12, activated charcoal manufactured by Mitsubishi Kasei K.K. (Diasorb G•6-10) (specific surface area 900 $m^2/g$), and various oxidizing agents shown in Table 3 were mixed to prepare deodorizer compositions (Present Products 1 - 12).

These products were evaluated by the evaluation methods shown below, and the evaluation results thereof are shown in Table 3 together with those of Comparative Examples.

Deodorizing Effect Evaluation Method A

Meat, vegetable, and fish were placed in a 6-liter desiccator and left to stand for 2 weeks to, prepare an artificial green garbage having an unpleasant odor.

A 5 g amount of the deodorizer composition was wrapped in an nonwoven cloth and placed in a wide mouth bottle to provide a sample. Further, from the 6-liter desiccator, 10 ml of the head space gas of the artificial green garbage odor was introduced into the wide mouth bottle, and the odor strength over a lapse of time was evaluated organoleptically, according to the following standards.

| Evaluation point | Evaluation standard |
|---|---|
| 0 | no odor |
| 1 | very slight odor |
| 2 | slight odor |
| 3 | pervasive odor |
| 4 | strong odor |
| 5 | very strong odor |

The evaluation was conducted by an expert panel of 5 members. The evaluations made by the top and bottom members were ignored, and an average of the evaluations made by the remaining three members was obtained by rounding off.

EP 0 376 448 B1

Table 3

| | | Deodorizer Composition | | | Evaluation results over lapse of time | | |
|---|---|---|---|---|---|---|---|
| | | Component (a) | Component (b) (oxidizing agent) | Formulation amount ($\%$) (a)/(b) | After 1 hr | After 3 hrs | After 6 hrs |
| Example (Present product) | 1 | Activated charcoal | Ammonium persulfate | 97/3 | 3 | 1 | 0 |
| | 2 | " | Potassium monopersulfate | 50/50 | 3 | 1 | 0 |
| | 3 | " 3 | Potassium persulfate | 95/5 | 1 | 0 | 0 |
| | 4 | " 4 | Magnesium·perphthalate | 3/97 | 1 | 0 | 0 |
| | 5 | " 6 | Diperoxy dodecane diacid | 55/45 | 1 | 0 | 0 |
| | 6 | " 7 | Potassium monopersulfate | 60/40 | 1 | 0 | 0 |

11

Example 2

A mixture of 90 g of the aluminosilicate obtained in Synthesis Example 1 and 10 g of potassium persulfate was kneaded with 50 g of water, by a mortar, followed by drying at 80°C to obtain a cake. The

Table 3 (Continued)

| | Deodorizer Composition | | | Evaluation results over lapse of time | | |
| --- | --- | --- | --- | --- | --- | --- |
| | Component (a) | Component (b) (oxidizing agent) | Formulation amount (z) (a)/(b) | After 1 hr | After 3 hrs | After 6 hrs |
| Example 7 (Present product) | Synthetic Example 9 | Sodium persulfate | 10/90 | 1 | 0 | 0 |
| 8 | " 10 | Ammonium persulfate | 30/70 | 1 | 0 | 0 |
| Comparative Example 1 | Activated charcoal | - | 100/0 | 4 | 3 | 2 |
| 2 | - | Calcium hypochlorite | 0/100 | 4 | 3 | 3 |
| 3 | - | - | - | 5 | 5 | 5 |

cake was crushed and sifted through a sieve to obtain a deodorizer composition as a white powder of 4 to 8 mesh (Present Product 15).

The results of the evaluation of this composition are shown below in Table 4, together with the results of Example 3.

Example 3

A mixture of 95 g of silica powder, 5 g of sodium percarbonate was kneaded with 50 g of water, by a mortar, followed by drying at 80°C to obtain a cake. The cake was crushed and sifted through a sieve to obtain a white powder of 4 to 8 mesh. The white powder and the aluminosilicate obtained in Synthesis Example 2 were mixed to a formulation ratio of 9:1 to obtain a deodorizer composition (Present Product 16).

The deodorizing effect was evaluated according to the evaluation method described in Example 1, and the results of the evaluations of Examples 2 and 3 are shown in Table 4.

Table 4

| Deodorizer composition | | Evaluation results over lapse of time | | |
|---|---|---|---|---|
| Component (a) | Component (b) (oxidizing agent) | After 1 hr | After 3 hrs | After 6 hrs |
| Present product — Synthetic Example 1 2 | Potassium persulfate | 1 | 0 | 0 |
| Comparative Example — Activated charcoal 4 | – | 4 | 3 | 2 |
| – 5 | – | 5 | 5 | 5 |

Example 4

A 2 g amount of dried leaves of rosemary was crushed by a cutter, charged into a Soxhlet extractor, and subjected to hot extraction with an addition of 180 g of water for 15 hours, whereby a 10% rosemary extract was obtained.

A 100 g amount of the aluminosilicate obtained in Synthesis Example 1 was impregnated with the 10% Rosemary extract and dried at 80 °C, and the cake thus obtained was sifted through a sieve of 8 to 16 mesh to obtain a deodorizer composition.

13

Example 5

A 20 g amount of the whole grass of thyme was crushed, charged into a Soxhlet extractor, and subjected to hot water extraction with an addition of 180 g of water for 13 hours to obtain an extract. A deodorizer composition was obtained in the same manner as in Example 4.

Example 6

A 10 g amount of leaves of dried sage was crushed, charged into a Soxhlet extractor, and subjected to hot extraction with an addition of 190 g of 50% ethanol for 15 hours to obtain a 5% sage extract. An amount of 100 g of the aluminosilicate obtained in Synthesis Example 2 was impregnated with 10 g of the 50% sage extract obtained by concentration by an evaporator, followed by drying at 110°C. The cake obtained was sifted through a sieve of 8 to 16 mesh to obtain a deodorizer composition.

Example 7

The 10% rosemary extract obtained in Example 4 was sprayed onto 100 g of the aluminosilicate obtained in Synthesis Example 3 to obtain a powdery deodorizer composition.

Example 8

The 10% rosemary extract obtained in Example 4 was sprayed onto activated charcoal (manufactured by Mitsubishi Kasei K.K.; Diasorb G-6-10) to obtain a powdery deodorizer composition.

Example 9

The sage extract obtained in Example 6 was sprayed onto spherical silica to obtain a deodorizer composition.

Example 10

A 30 g amount of pine leaves was crushed, charged into a Soxhlet extractor, and subjected to hot extraction with an addition of 150 g of acetone for 10 hours to obtain an extract. A deodorizer composition was the obtained by the same treatment as used in Example 4.

Evaluation of the Deodorizing Effect (Examples 4 - 10)

The deodorizer compositions of Examples 4 to 10 were evaluated according to the above-mentioned evaluation method A.

14

The results are shown in Table 5.

## Table 5

| | Sample | Deodorizing effect | | |
|---|---|---|---|---|
| | | 1 hr | 3 hrs | 6 hrs |
| Present product | Example 4 | 1 | 0 | 0 |
| | " 5 | 2 | 1 | 0 |
| | " 6 | 1 | 0 | 0 |
| | " 7 | 1 | 0 | 0 |
| | " 8 | 3 | 1 | 0 |
| | " 9 | 3 | 1 | 0 |
| | " 10 | 2 | 1 | 0 |
| Comparative Example | 6 (10% Rosemary extract) | 4 | 4 | 3 |
| | 7 (Activated charcoal) | 4 | 3 | 3 |
| | 8 (50% Sage extract) | 4 | 4 | 3 |
| | 9 (Blank) | 5 | 5 | 5 |

Examples 11 - 18

The aluminosilicates obtained in Synthesis Examples 1 to 3, activated charcoal manufactured by Mitsubishi Kasei K.K. (Diasorb G-6-10), and various sterilizers shown in Table 6 were mixed to prepare deodorizer compositions.

These were evaluated according to the following evaluation method (method B), and the evaluation results thereof are shown in Table 6 together with those of Comparative Examples.

Deodorizing Effect Evaluation Method B

Meat, vegetables, fish, were placed in a 6-liter desiccator and left to stand for one week, to prepare an artificial green garbage. An amount of 30 g of the green garbage prepared was placed into a wide mouth bottle of 1.8-liter, 10 g of the deodorizer composition sprayed thereon, and the odor intensity over a lapse of time was evaluated organoleptically according to the following standards.

15

| Evaluation point | Evaluation standard |
|---|---|
| 0 | no odor |
| 1 | very slight odor |
| 2 | slight odor |
| 3 | pervasive odor |
| 4 | strong odor |
| 5 | very strong odor |

Evaluation was conducted by an expert panel of 5 members. The top and bottom members were ignored, and an average of the evaluations of the remaining three members was obtained by rounding off.

EP 0 376 448 B1

Table 6

| | | Deodorizer Composition | | | Evaluation results over lapse of time | | | | |
|---|---|---|---|---|---|---|---|---|---|
| | | Component (a) | Component (b) (Germicide) | Formulation ratio (a)/(b) | After 1 hr | After 3 hrs | After 6 hrs | After 1 day | After 2 days |
| Example (Present product) | 11 | Activated charcoal | Benzalkonium chloride | 99/1 | 3 | 1 | 0 | 0 | 0 |
| | 12 | " | o-Phenylphenol | 99.9/0.1 | 2 | 1 | 0 | 0 | 0 |
| | 13 | " | 2-(4-Thiazolyl)benzimidazole | 50/50 | 2 | 1 | 0 | 0 | 0 |
| | 14 | Synthetic Example 3 | p-Chloro-m-xylenol | 95/5 | 1 | 0 | 0 | 0 | 0 |
| | 15 | " 2 | α-Bromocinnamic aldehyde | 97/3 | 2 | 1 | 0 | 0 | 0 |

Table 6 (Continued)

| | | Deodorizer Composition | | Evaluation results over lapse of time | | | | |
| | Component (a) | Component (b) (Germicide) | Formulation ratio (a)/(b) | After 1 hr | After 3 hrs | After 6 hrs | After 1 day | After 2 days |
|---|---|---|---|---|---|---|---|---|
| Comparative Example 10 | Activated charcoal | - | 100/0 | 4 | 3 | 2 | 2 | 3 |
| 11 | - | Benzalkonium chloride | 0/100 | 4 | 3 | 3 | 3 | 3 |
| 12 | Activated charcoal | Methyl p-oxybenzoate | 99.995/0.005 | 4 | 3 | 2 | 2 | 2 |
| 13 | Silica powder | - | 100/0 | 4 | 3 | 3 | 3 | 4 |
| 14 | - | - | - | 5 | 5 | 5 | 5 | 5 |

Example 16

A 100 g amount of the aluminosilicate obtained in Synthesis Example 3 was impregnated with 30 g of 10% ethyl alcohol solution of o-phenylphenol, and dried at 80°C for 30 minutes to obtain a powdery

18

deodorizer composition.

When the deodorizer composition was sprayed into a polymer bucket and a triangular sink-corner container for garbage, containing green garbage, the odor generated from the green garbage or remaining foods was eliminated.

Examples 17 - 20

Samples of each of the wastewaters shown below were placed, to an amount of one liter, in a 1.8-liter glass bottle.

Wastewater-A:     washed wastewater from pig sty (feeding 87 pigs)

Wastewater-B:     wastewater from cleaning tank of a standard family having 4 members.

Then 10 g of the deodorizer composition shown in Table 5 was added thereto, and the odor intensity over a lapse of time was organoleptically evaluated. The evaluation standards were made according to method B.

The deodorizer composition was a powdery product obtained by impregnating the aluminosilicate obtained in each Synthesis Example with an ethanolic solution of the sterilizer, followed by drying at 80°C for one hour, and supplemented with 1% of sterilizer.

Table 7

| Deodorizer composition | | Treated waste water | Evaluation results over lapse of time | | | | |
|---|---|---|---|---|---|---|---|
| Component (a) | Component (b) (Germicide) | | After 1 hr | After 3 hrs | After 6 hrs | After 1 day | After 2 days |
| Example 17 | Synthetic Example 2 | p-chloro-m-xylenol | A | 2 | 1 | 0 | 0 | 0 |
| " 18 | " 3 | o-phenylphenol | B | 2 | 1 | 0 | 0 | 0 |
| " 19 | " 2 | o-phenylphenol | A | 2 | 1 | 0 | 0 | 0 |
| " 20 | " 2 | Butyl m-cresol | A | 2 | 1 | 0 | 0 | 0 |
| Comparative Example 17 | Silica | - | B | 4 | 3 | 3 | 3 | 4 |
| 18 | - | Dehydroacetic acid | B | 4 | 3 | 3 | 3 | 3 |
| 19 | Activated charcoal | - | A | 4 | 3 | 3 | 3 | 4 |

Examples 21 - 23

Onto 10 g of the aluminosilicate obtained in Synthesis Examples 1 - 3, 2 g of the 10% rosemary extract was sprayed and dried at 80°C for 3 hours, and the sample obtained was mixed with 0.3 g of a sterilizer to

20

obtain a deodorizer composition.

The evaluations were made according to the evaluation method (method B), and the evaluation results are shown in Table 9 together with Comparative Examples.

Table 8

| | Deodorizer composition | | | Evaluation results over lapse of time | | | | |
|---|---|---|---|---|---|---|---|---|
| | Component (a) | plant extract (b)(ii) | Germicide (b)(iii) | After 1 hr | After 3 hrs | After 6 hrs | After 1 day | After 2 days |
| Example 21 | Synthetic 3 Example | Rosemary | o-phenylphenol | 2 | 1 | 0 | 0 | 0 |
| 22 | " 1 | " | Benzalkonium chloride | 2 | 1 | 0 | 0 | 0 |
| 23 | " 2 | " | Dehydroacetic acid | 2 | 1 | 0 | 0 | 0 |
| Comparative Example 20 | — | — | Benzalkonium chloride | 4 | 3 | 2 | 2 | 3 |
| 21 | Activated charcoal | — | — | 4 | 3 | 3 | 3 | 3 |
| 22 | — | Rosemary | Dehydroacetic acid | 3 | 2 | 2 | 2 | 2 |

**Claims**

1.  A deodorizer composition comprising:
    (a) an aluminosilicate having the following composition, in terms of oxides, represented by the composition ratio of the three components:
    $SiO_2$ : 5 to 80 mole%;
    $MO_{n/2}$: 5 to 65 mole%;
    $Al_2O_3$ : 1 to 60 mole%;
    wherein M represents at least one metal selected from the group consisting of zinc, copper, silver, cobalt, nickel, iron, titanium, barium, tin and zirconium, and n represented a valence of metal; and
    (b) at least one component selected from the group consisting of (i) at least one oxidizing agent selected from the group consisting of organic peracids and salts thereof and persulfate, (ii) at least one plant extract selected from the group consisting of Rosemary extracts and Sage extracts, and (iii) at least one germicide selected from the group consisting of p-chloro-m-xylenol, butyl-m-cresol, o-phenylphenol, and $\alpha$-bromocinnamic aldehyde.

2.  A deodorizer composition as claimed in claim 1, wherein the weight ratio of the components (a)/(b) is from 1/99 to 99.99./0.01.

**Patentansprüche**

1.  Desorierendes Mittel, enthaltend (a) ein Aluminosilikat mit der folgenden Zusammensetzung in Verhältnissen von Oxyden, dargestellt druch das Zusammensetzverhältnis der drei Kompenenten:
    SiO2 : 5 bis 80 Molekular %;
    MOn/2 : 5 bis 65 Molekular %;
    Al2O3 : 1 bis 60 Molekular %;
    worin M wenigstens ein Metall aus der Gruppe bestehend aus Zink, Kupfer, Silber, Koblat, Nickel, Eisen, Titan, Barium, Zinn, und Zirkong und n eine Metallvalenz darstellt und
    (b) wenigstens eine Komponente aus der Gruppe bestehend aus (i) wenigstens einer oxydierenden Agens aus der Gruppe bestehend aus organischen Persäuren und deren Salzen und Persulfaten, (ii) wenigstens einem Agregatextrakt aus der Gruppe bestehend aus Rosmmarin-Extrakten und Salbei-Extrakten, und (iii) wenigstens ein keimtötende Substanz aus der Gruppe bestehend aus p-chloro-m-Xylenol, Butyl-m-Kresol, o-Phenylphenol, und d-Brimcinnamye-Aldehyd.

2.  Desodorierendes Mittel nach Anspruch 1, in dem das Gewichtsverhältnis der Komponenten (a)/(b) zwischen 1/99 und 99,99/0,01 liegt.

**Revendications**

1.  Composition désodorisante comprenant :
    (a) un aluminosilicate ayant la composition suivante, en termes d'oxydes, représentée par les proportions de composition des trois composants :
    $SiO_2$ : 5 à 80 % molaires ;
    $MO_{n/2}$ : 5 à 65 % molaires ;
    $Al_2O_3$ : 1 à 60 % molaires ;
    où M représente au moins un métal choisi parmi le zinc, le cuivre, l'argent, le cobalt, le nickel, le fer, le titane, le baryum, l'étain et le zirconium, et n représente une valence du métal ; et
    (b) au moins un composant choisi parmi (i) au moins un agent oxydant choisi parmi les peracides organiques et leurs sels et un persulfate, (ii) au moins un extrait végétal choisi parmi les extraits de romarin et les extraits de sauge, et (iii) au moins un germicide choisi parmi le p-chloro-m-xylénol, le butyl-m-crésol, l'o-phénylphénol et l'aldéhyde $\alpha$-bromocinnamique.

2.  Composition désodorisante selon la revendication 1, dans laquelle la proportion pondérale des composants (a)/(b) est de 1/99 à 99,99/0,01.